# EUROPÄISCHE PATENTSCHRIFT

(11) **EP 2 225 054 B1**
(45) Veröffentlichungstag und Bekanntmachung des Hinweises auf die Patenterteilung: **22.05.2013**
(21) Anmeldenummer: 08866172.3
(22) Anmeldetag: 29.12.2008
(51) Int. Cl.: B21C 1/00, B21C 23/00, B21C 37/04, A61L 17/06

(54) **VERFAHREN ZUR HERSTELLUNG EINES ELEMENTS AUS EINEM MAGNESIUMWERKSTOFF UND SO HERGESTELLTES ELEMENT**
METHOD FOR MANUFACTURING AN ELEMENT FROM MAGNESIUM MATERIAL AND RESULTING ELEMENT
PROCÉDÉ DE FABRICATION D'UN ÉLÉMENT EN UN MATÉRIAU À BASE DE MAGNÉSIUM, ET ÉLÉMENT AINSI FABRIQUÉ

(30) Priorität: 28.12.2007 DE 102007063317
(43) Veröffentlichungstag der Anmeldung: 08.09.2010
(73) Patentinhaber: Kucharski, Rafael, 30419 Hannover (DE)
(72) Erfinder: BACH, Friedrich-Wilhelm, 30916 Isernhagen (DE); KUCHARSKI, Rafael, 31135 Hildesheim (DE); BORMANN, Dirk, 30926 Seelze (DE)
(86) Internationale Anmeldenummer: PCT/EP2008/011125
(87) Internationale Veröffentlichungsnummer: WO 2009/083250

(56) Entgegenhaltungen:
- DE-A1- 10 128 100
- DE-C- 630 061
- DE-C- 665 836
- GB-A- 425 316
- US-A- 2 750 311
- DATABASE WPI Thomson Scientific, London, GB; AN 1989-191190 XP002521040 -& SU 1 447 462 A (INST METALLURGII IMENI) 30. Dezember 1988 (1988-12-30)
- DATABASE WPI Thomson Scientific, London, GB; AN 2005-197200 XP002521041 -& CN 1 554 495 A (UNIV DALIAN SCIENCE & ENG) 15. Dezember 2004 (2004-12-15)

## Beschreibung

Die vorliegende Erfindung richtet sich auf ein Verfahren zur Herstellung von Elementen aus einem Magnesiumwerkstoff, insbesondere auf die Herstellung von Drahtmaterial aus einer Magnesiumlegierung, wobei das hergestellte Drahtmaterial bevorzugt einen Durchmesser von 0,5 mm oder kleiner aufweist. Das Drahtmaterial setzt sich dabei zumindest aus mehreren einzelnen Halbzeugen wie Drähten einer Magnesiumlegierung zusammen, die als Litze bzw. Seil zusammengelegt und weiter mittels Ziehen umgeformt werden. Die vorliegende Erfindung richtet sich weiterhin auf Drahtmaterial, das mit dem erfindungsgemäßen Verfahren hergestellt ist. Bei diesem Drahtmaterial handelt es sich bevorzugt um resorbierbares Nahtmaterial.

### Stand der Technik

Nahtmaterial oder Klammermaterial zum Zusammenfügen von gleichen oder verschiedenen Gewebearten bei Tieren einschließlich Menschen muss verschiedenste Anforderungen erfüllen.

Einerseits muss das Material soweit inert sein, dass in den lebenden Organismen keine Abstoß- oder Entzündungsreaktionen hervorgerufen werden oder die Materialien einen negativen Einfluss auf die Gesundung des Gewebes oder des gesamten Organismus hervorrufen.

Andererseits müssen die Materialien unterschiedlichsten mechanischen Anforderungen genügen. So muss das Material eine ausreichende Zugfestigkeit aufweisen, um zum Beispiel ein Zusammenhalten des Gewebes zu gewährleisten. Das Material darf des Weiteren nicht zu spröde sein, um ein Brechen bei Belastung zu verhindern. Schließlich muss das Material eine ausreichende Biegsamkeit aufzeigen, um sich gut dem Gewebe anzupassen und um deformierbar, z.B. zur Knotenbildung, zu sein, ohne dass das Material über die Zeit seine Form verliert. Das heißt, das Material darf sich weder unter Last weiter strecken noch aufgrund von Verformung ein Öffnen der Gewebewunde ermöglichen.

Das Nahtmaterial kann sowohl als permanentes Material, das heißt das Material wird über eine bestimmte Zeit nicht abgebaut, oder in absorbierbarer Form verwendet werden. Dabei ist die Nachfrage nach absorbierbarem Material sehr groß, insbesondere, um dieses Material im Körper des Tiers einschließlich des Menschen als Nahtmaterial einzusetzen.

Absorbierbares Material, insbesondere Magnesium enthaltendes absorbierbares Material, ist aus dem Stand der Technik bekannt. So beschreibt die EP 1395297 medizinische Implantate für den menschlichen oder tierischen Körper aus Magnesiumlegierungen, die im Körper degradieren. Die dort beschriebenen Materialien sollen die im Stand der Technik bekannten Nachteile bei der Verwendung absorbierbaren Materials aus Magnesiumlegierungen überwinden. Diese Nachteile beinhalten eine relativ große Gasmengenproduktion pro Zeiteinheit insbesondere von Wasserstoff. Dadurch entstehen Gaskavernen im Körper und die Materialien selbst werden ungleichmäßig degradiert.

Die DE 10 2004 036 399 beschreibt Naht- und Klammermaterial, das sich dadurch auszeichnet, dass es große Mengen Seltenerdmetalle und Yttrium enthält. Bevorzugt soll dabei die Magnesiumlegierung kein Aluminium enthalten. Die Art und Weise der Herstellung des Naht- und Klammermaterials ist in der DE 10 2004 036 399 nicht beschrieben, gleiches gilt für die EP 1395297.

Die Idee der Verwendung von Magnesium und Magnesiumlegierungen als bioresorbierbare Materialien, z.B. als Nahtmaterial, ist aber schon über 70 Jahre alt. So wird chirurgisches Nahtmaterial aus Magnesium oder Magnesiumlegierungen bereits in den deutschen Patentschriften DE 630061, DE 676059, DE 665836 und DE 688616 beschrieben. Diese dort beschriebenen Nahtmaterialien wiesen aber große Nachteile hinsichtlich der Gasentwicklung und des ungleichmäßigen Korrosionsangriffs auf.

Die Herstellung der resorbierbaren Materialien wie Nahtmaterialien aus Magnesiumlegierungen erfordert eine Vielzahl von Prozessschritten der plastischen Bearbeitung von Metallen. Diese Umformungsprozesse der Metalle werden von vielen Parametern beeinflusst, wie zum Beispiel der Temperatur sowohl des Umformungswerkzeugs als auch des zu umformenden Gegenstandes bei der Umformung.

In der DE 630061 wird z. B. ein Herstellungsverfahren für dünne Magnesiumdrähte in mehreren Schritten mit zusätzlicher Wärmebehandlung beschrieben.

Der folgenden Erfindung liegt die Aufgabe zugrunde, degradierbare Elemente, wie Drahtmaterial, und insbesondere Nahtmaterial für chirurgische Zwecke, bereitzustellen. Dabei soll neben der Biokompatibilität das Material eine ausreichende Zugfestigkeit aufweisen und die notwendige Biegsamkeit besitzen.

### Beschreibung der Erfindung

Diese Aufgabe wird durch das erfindungsgemäße Verfahren zur Herstellung von Elementen, wie Drahtmaterial (bei dem als Halbzeug Drähte verwendet werden), aus einem Magnesiumwerkstoff gelöst. Dieses Verfahren eignet sich zur Herstellung von Drahtmaterial aus Magnesiumwerkstoffen, wobei das erhaltene Drahtmaterial nach der Herstellung bevorzugt einen Durchmesser von 0,5 mm oder kleiner aufweist.

Das erfindungsgemäße Verfahren zur Herstellung eines Elements aus einem Magnesiumwerkstoff wie einem Drahtmaterial umfasst die Schritte:
a) Warmstrangpressen eines Magnesiumwerkstoffes unter Erhalt eines Strangmaterials;
b) eine erste Wärmebehandlung des im Schritt a) erhaltenen Strangmaterials;
c) ein erstes Ziehen des im Schritt b) wärmebehandelten Strangmaterials unter Erhalt eines Halbzeugs eines Magnesiumwerkstoffes; und gegebenenfalls Wiederholen dieser Schritte
d) Legen einer Litze umfassend mindestens zwei der im Schritt c) erhaltenen Halbzeuge eines Magnesiumwerkstoffes;
e) Ziehen der im Schritt d) gelegten Litze, wobei dieses Ziehen ein warmes und/oder kaltes Ziehen ist und f) ggf. ein sich an den Schritt e) anschließendes abschließendes Kaltziehen.

Bei Durchführung des erfindungsgemäßen Verfahrens werden drei Hauptparameter für Elemente aus Magnesiumwerkstoffen, insbesondere für Nahtmaterialien; nämlich die mechanischen Eigenschaften, das Gefüge, aber auch die Geometrie des Elements vorteilhaft verbessert.

Vorliegend wird unter Halbzeug ein einstückiges Werkstück verstanden, während ein Drahtmaterial mehrere Einzeldrähte umfassen kann. Der Begriff Element, wie er vorliegend verwendet wird, umfasst Drahtmaterialien und kann aus zwei oder mehreren Einzelbestandteilen also Halbzeugen, zusammengesetzt sein.

Mit Hilfe des erfindungsgemäßen Verfahrens wird insbesondere ein Drahtmaterial z.B. ein Nahtmaterial erhalten, das im Gegensatz zu einem Einzeldraht in seiner Geometrie vorteilhaft ausgebildet ist. Des Weiteren weisen die mit dem erfindungsgemäßen Verfahren hergestellten Materialien überragende Zugfestigkeiten auf, ohne in ihrer Biegsamkeit oder Duktilität eingeschränkt zu sein.

Gemäß dem erfindungsgemäßen Verfahren wird in einem ersten Schritt ein Magnesiumwerkstoff einem Strangpressen unterworfen. Hierbei handelt es sich um ein Warmstrangpressen mit dem zum Beispiel ein Halbzeug mit einer Dicke zwischen 1,0 mm und 1,5 mm erhalten wird.

In diesem Zusammenhang umfasst der Ausdruck Magnesiumwerkstoff sowohl solche aus reinem Magnesium als auch Magnesiumlegierungen.

Bei dem direkten Warmstrangpressen mit Matrize wird eine Flachmatrize, die die Form des Halbzeugs bestimmt, und ein Stempel mit etwas geringerem Durchmesser als der Innendurchmesser des Rezipienten verwendet. Beim direkten Warmpressen bildet sich deshalb zwischen Pressscheibe und Rezipientenwandung eine Werkstoffschale. Von Vorteil ist hierbei, dass beim Pressen Oxidreste und verunreinigte Blockaußenzonen in diese Schale gelangen und so nicht in das Pressprodukt einfließen können. Darüber hinaus bildet sich beim Pressvorgang eine so genannte tote Zone vor dem Werkszeug aus, da durch starke Reibung zwischen Pressblock und Blockhalterung die äußere Schicht des Presslings gegenüber dem Blockinneren zurückversetzt bleibt.

Durch die Bildung von Werkstoffschale und toter Zone werden metallische, saubere Profiloberflächen gewährleistet.

Beim Strangpressen finden verschiedene dynamische Erholungs- und Kristallisationsvorgänge sowohl vor als auch in der Umformzone statt. Diese Kristallisationen umfassen sowohl dynamische als auch statische Kristallisationen als auch die entsprechenden Erholungen unter Abbau des Verfestigungszustandes und Gitterfehlern.

Durch die Warmumformung wird das grobe Gussgefüge in ein feinkörniges, lang gestrecktes Gefüge umgewandelt. Eine solche Kornfeinung ist mit einer deutlichen Verbesserung der Werkstoffkennwerte der jeweiligen Magnesiumlegierung verbunden. Die erreichbaren Werkstoffkennwerte verbessern sich hierbei mit zunehmender Stärke des Pressens, das heißt mit zunehmender Durchknetung.

Anschließend wird im nächsten Schritt des erfindungsgemäßen Verfahrens das stranggepresste Material, das Strangmaterial, einer Wärmebehandlung unterworfen. Dabei sollen dem zu behandelnden Werkstoff möglichst optimale Eigenschaften verliehen werden. Bei der Rekristallisation werden Keimbildung und Wachsen neuer Kristallite (primäre Rekristallisation) eingeleitet, wodurch ein schneller Erholungsprozess mit Aufhebung innerer Spannungen und Erzeugung polygonaler Subkorngrenzen initiiert wird. An diesen Grenzen bilden sich neue kleine Kristallite, wobei Versetzungen weitgehend abgebaut werden. Wegen der stark reduzierten Versetzungsdichte besitzen rekristallisierte Metalle an sich nur geringe Festigkeit, aber hohe Duktilität.

Der erste Schritt der Wärmebehandlung dient der Erholung des Materials sowie dem Abbau von inneren Spannungen.

Dem Schritt der Wärmebehandlung schließt sich ein erstes Ziehen (Drahtziehen) des wärmebehandelten Strangmaterials an. Das Ziehen als eines der bekannten Umformverfahren erlaubt den Erhalt einer Oberflächenbeschaffenheit und Oberflächengüte des Halbzeugs, sowie von geometrischen Eigenschaften des Halbzeugs, wie sie z. B. für die Verwendung als Nahtmaterial benötigt werden. Deshalb eignet sich das Verfahren des Ziehens besonders für den Einsatz zur Herstellung von sehr dünnen Halbzeugen (Drähten) und Drahtmaterialien aus einem Magnesiumwerkstoff mit Durchmessern von unter 1 mm, wie 0,5 mm und geringer, insbesondere 0,3 mm und geringer. Bei Nahtmaterialien ist es z. B. notwendig, möglichst glatte Oberflächen für eine hinreichende Biokompatibilität zu erreichen, wobei die notwendigen mechanischen Eigenschaften nicht verschlechtert werden dürfen. Das Ziehen ist als Warm- oder Kaltziehen möglich.

Beim Warmziehen oder Warmumformen können die Temperaturen in der Werkzone aus Werkstück/Werkzeug sehr hohe Werte erreichen. Selbst beim Kaltziehen können kurzzeitig hohe Temperaturen auftreten, denn mehr als 80% der für den Umformvorgang aufzubringenden Energie werden in Wärme umgesetzt und führen zusammen mit der Reibungsarbeit zu einer Erwärmung von Werkstück und Werkzeug. Durch diese Erwärmung kann es möglich sein, dass im Werkstück wie beim Strangpressen Erholungs- und Rekristallisationsvorgänge ablaufen. Die geringe Fläche des Halbzeugs, als auch des Drahtmaterials (Drahtverbund) garantieren eine schnelle Aufwärmphase als auch eine schnelle Kühlungsphase.

Erfindungsgemäß umfasst das Ziehen des wärmebehandelten Strangmaterials vorzugsweise zwei, wie drei, vier, fünf, sechs, sieben, acht, neun, zehn usw. einzelne Ziehschritte. Dabei wird mindestens beim ersten Durchgang des Ziehens ein Kaltziehen durchgeführt. Hier wird das erwärmte Halbzeug über einen nicht erwärmten Ziehstein bzw. mit Hilfe einer nicht erwärmten Ziehdüse auf einen reduzierten Halbzeugdurchmesser gebracht. Anschließend wird das Halbzeug erneut erwärmt, bevorzugt findet diese Erwärmung derart statt, dass das Material erweicht wird, ein so genanntes Entspannungsglühen. Im Falle von Magnesiumwerkstoffen liegt der Bereich der Erwärmung bei 150°C bis 350°C, bevorzugt 200°C bis 300°C, je nach Art des Werkstoffes. Im Falle des Kaltziehens wird das Halbzeug zum Beispiel vorher auf eine Temperatur von 270°C bis 330°C, wie 300°C erwärmt, um anschließend kaltgezogen zu werden. Dieser Vorgang des Kaltziehens wird mindestens einmal, bevorzugt mehrere Male wiederholt. Anschließend wird das Halbzeug erwärmt und ggf. unter Ausschluss von Sauerstoff, wie unter Schutzgas, zum Beispiel Argon, mit einem erwärmten Ziehstein gezogen. Nach erneutem Erwärmen wird das Halbzeug ggf. ein zweites Mal warmgezogen. Bevorzugt wird der Halbzeug dabei auf einen Durchmesser von 0,5 mm oder kleiner, wie 0,3 mm oder kleiner, gezogen.

Aus der Literatur ist bekannt, dass sich durch eine externe Wärmezufuhr beim Ziehen die Dehnung der Werkstoffe vergrößern lässt, wobei sich gleichzeitig die Ziehkraft verringert. In diesem Fall ist es auch möglich, mehrere Zyklen durchzuführen, ohne den zeitaufwendigen Wärmebehandlungsprozess in Anspruch nehmen zu müssen. Nachteil der Vorerwärmung des Materials ist eine starke Oberflächenoxidation; die Kühlung des Formmaterials ist stark von dessen Querlänge abhängig. Bevorzugt wird daher das Material unmittelbar vor dem Ziehstein im laufenden Ziehprozess erwärmt.

Zu hohe Temperatur, zu hohe Ziehgeschwindigkeit oder eine zu starke Erwärmung der Ziehsteine führt allerdings zu verschiedensten Ziehdefekten.

Die erhaltenen Halbzeuge der Magnesiumwerkstoffe werden in einem weiteren Schritt zu Litzen oder Seilen gelegt, wobei mindestens zwei Halbzeuge zu einer Litze bzw. Seil zusammengefügt werden. Gegebenenfalls erfolgt ein Verdrehen der Halbzeuge. Dieses erhaltene Seil bzw. Litze wird dann einem weiteren Ziehen unterworfen, um schließlich eine Litze bzw. Seil als erfindungsgemäßes Element, insbesondere als Drahtmaterial mit einem Durchmesser von 0,5 mm oder kleiner, zu erhalten. Dazu wird z. B: in dem Ziehschritt die Litze erwärmt und im erwärmten Zustand mit einem warmen Ziehstein in seinem Durchmesser reduziert. Die Reduzierung des Durchmessers erfolgt dabei bevorzugt in mehreren Schritten. Alternativ kann das Ziehen ein Kaltziehen mit einem vorherigen Erholungsglühen sein.

Dieser Vorgang des Warmziehens wird solange durchgeführt, bis der gewünschte Durchmesser erreicht wird. Bevorzugt findet dabei dieses Ziehen unter Sauerstoffausschluss insbesondere unter Schutzgasatmosphäre statt, um ein Oxidieren der Oberfläche zu verhindern.

Abschließend kann das so erhaltene Element gegebenenfalls einem weiteren Kaltziehen unterworfen werden, um ein Element mit dem gewünschten Durchmesser von insbesondere 0,5 mm oder kleiner zu erhalten.

Mit dem erfindungsgemäßen Verfahren ist es möglich, Drahtmaterial mit sehr kleinem Durchmesser von unter 0,5 mm, wie 0,3 mm oder kleiner, zu erhalten. Solche Dimensionen sind insbesondere für resorbierbare Nahtmaterialien geeignet. In einer Ausführungsform betrifft die vorliegende Erfindung daher ein Verfahren zur Herstellung von Drahtmaterialien, insbesondere Nahtmaterialien auf Basis eines Magnesiumwerkstoffes, wobei der Durchmesser des Drahtmaterials 0,3 mm oder kleiner ist.

Bevorzugt weisen die Seile bzw. Litzen dabei 3, 4, 5, 6, 7 oder 8 Einzelhalbzeuge (Einzeldrähte) der nach dem ersten Ziehen erhaltenen Magnesiumwerkstoffhalbzeuge auf. Die Anzahl und Stärke der Einzeldrähte hängt auch von dem zu verbindenden Geweben (Hart- oder Weichgewebe) ab.

Besonders bevorzugt ist, dass die Reduktion des Durchmessers beim Ziehen nicht größer als 0,05 mm ist. Dadurch wird eine Homogenität des Materials gewährleistet und eine gute Zugfestigkeit bei gleichzeitig Verbesserung der Biegsamkeit erzielt.

In einer bevorzugten Ausführungsform ist das Verfahren zur Herstellung von resorbierbaren Drahtmaterialien, insbesondere resorbierbaren Nahtmaterials oder Klammermaterial, geeignet. Aufgrund des erfindungsgemäßen Verfahrens weist das erhaltene Nahtmaterial hervorragende geometrische als auch mechanische Eigenschaften auf. Die Form des erfindungsgemäß hergestellten Elements kann dabei den gewünschten Eigenschaften angepasst sein. Eine möglichst große Oberfläche des Materials erlaubt z. B. eine schnellere Resorption als eine kompakte, wie z. B. runde Form. Vorliegend werden die Ausdrücke "Resorption" und Absorption" synonym verwendet.

Als Ausgangsmaterial für das erfindungsgemäße Verfahren sind insbesondere Solche Magnesiumwerkstoffe geeignet, die neben Magnesium auch Aluminium und Lithium enthalten. Gegebenenfalls kann diese Magnesiumlegierung weiterhin Zirkonium enthalten. Bevorzugt liegen die Mengen an Aluminium, Lithium und ggf. Zirkonium in den folgenden Bereichen:

| | |
|---|---|
| Al | 0 bis 2 Massen-% |
| Mn | 0 bis 1 Massen-% |
| Zn | 0 bis 2 Massen-% |
| Li | 0 bis 9 Massen-% |
| Zr | 0 bis Massen-5% |
| Seltene Erden | 0 bis 2 Massen-% |

Es zeigte sich insbesondere, dass bevorzugt die Magnesiumlegierung kein Kalzium enthält, da Kalzium einen negativen Einfluss auf die Zugfestigkeit aufweist. Besonders bevorzugte Legierungen sind: MgAl₃Li₉; LAE442; oder MgAl₃Li₆.

Die in der Litze bzw. Seile vorhandenen Magnesiumhalbwerkzeuge (Drähte) können dabei gleichen Durchmessers oder unterschiedlichen Durchmessers sein. In einer bevorzugten Ausführungsform werden 2, 3, 4, 5, 6, 7, 8 oder 9 Magnesiumdrähte gleichen Durchmessers zur Herstellung der Litze bzw. des Seils verwendet.

In einer weiteren Ausführungsform betrifft die vorliegende Erfindung Elemente, z.B. Drahtmaterialien, die mit dem erfindungsgemäßen Verfahren hergestellt sind. Bei diesen Elementen, wie Drahtmaterialien handelt es sich insbesondere um Nahtmaterialien, wie resorbierbare Nahtmaterialien aus Magnesiumwerkstoffen.

Die Elemente können auch zu anderen Gegenständen weiterverarbeitet werden. So kann das erhaltene Element in resorbierbaren oder permanenten Implantaten eingesetzt werden. Es ist zum Beispiel ein Implantat zur Förderung des Knochenaufbaus oder von anderen Geweben, wie Muskelgeweben, z. B. Herzmuskelgewebe möglich.

Wenn es sich bei den verwendeten Magnesiumwerkstoffen um permanente Magnesiumlegierungen handelt, so können die erfindungsgemäß erhaltenen Elemente auch Ligaturclips oder andere in der Medizintechnik eingesetzte beständige Gegenstände und Instrumente sein. Andere Verwendungen von permanenten Elementen sind z.B. Kettenhemden und andere Produkte, die einerseits eine Stabilität aber auch Flexibilität erfordern, ohne dass eine zu starke Dehnung auftritt.

Die Elemente können bei den oben genannten Anwendungen als Elemente als solche, d. h. Litzen oder Seile, oder in einer weiterverarbeiteten Form, z.B. durch flechten, weben, etc. eingesetzt werden.

Es zeigte sich, dass die mit dem erfindungsgemäßen Verfahren hergestellten Elemente mit einem ausreichend geringem Durchmesser herstellbar sind, wobei die notwendigen mechanischen Eigenschaften für z. B. Nahtmaterialien vorliegen, das heißt eine hohe Zugfestigkeit bei guter Biegsamkeit.

Die erfindungsgemäßen Elemente können dabei rund als auch flach ausgebildet sein.

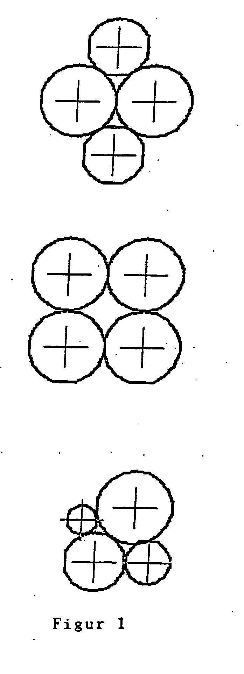

In den beigefügten Abbildungen 1 bis 5 sind verschiedene Ausführungsformen des erfindungsgemäß erhältlichen Elements im Querschnitt dargestellt. In der Abbildung 1 sind verschiedene Ausführungsformen eines Elements mit vier Einzeldrähten (Halbzeugen) dargestellt. Dargestellt sind mögliche Geometrien dieses Elements. Die linke und rechte Abbildung zeigen Beispiele für ein Element mit Einzeldrähten unterschiedlichen Durchmessers, während die mittlere Abbildung ein Beispiel für ein Element aus vier Drähten gleichen Querschnitts darstellt.

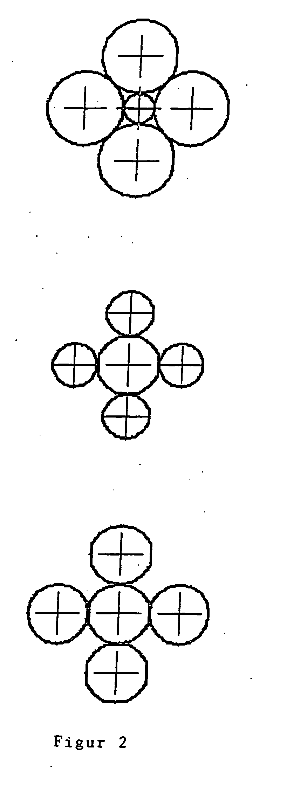

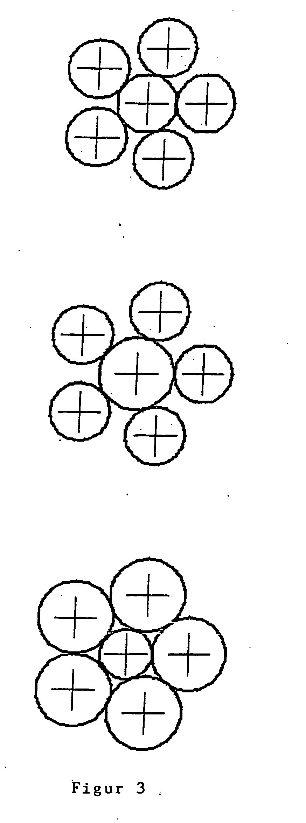

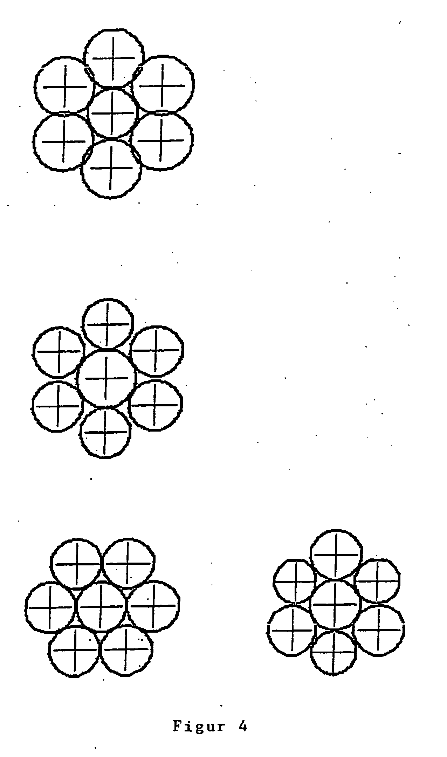

Abbildung 2 zeigt ein Beispiel für ein Element aus fünf Einzeldrähten. Die mittlere und rechte Abbildung zeigt ein Beispiel für Drähte unterschiedlichen Durchmessers, während die linke Abbildung ein Beispiel für ein erfindungsgemäßes Element aus Drähten mit gleichem Durchmesser darstellt. Die in der Abbildung 2 dargestellte Geometrie kann im Hinblick auf eine gute Resorbierbarkeit und Korrosion des Materials im Körper aufgrund der großen Oberfläche vorteilhaft sein. Die Abbildung 3 stellt ein Beispiel für erfindungsgemäße Elemente aus sechs Einzeldrähten dar. In der Abbildung 4 sind beispielhafte Querschnitte für erfindungsgemäße Elemente aus sieben einzelnen Drähten dargestellt, wobei bei Verwendung von Drähten unterschiedlichen Durchmessers verschiedene Oberflächenformen erhalten werden.

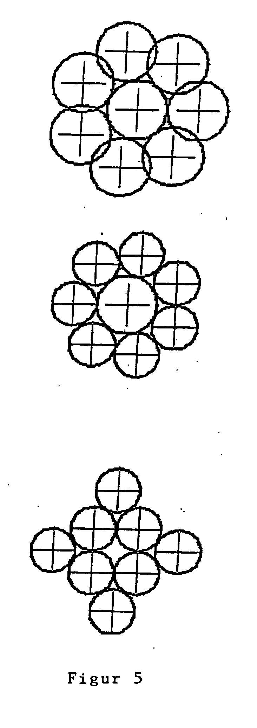

In Abbildung 5 werden schließlich beispielhaft Querschnitte für erfindungsgemäße Elemente aus acht Einzeldrähten dargelegt. Hierbei kann die Anordnung der Drähte sowohl in einer möglichst runden Form erfolgen, siehe die linke und mittlere Ausführungsform der Figur 5. Eine Ausführungsform, wie auf der rechten Seite der Abbildung 5 dargestellt, mit einer sternenförmigen Anordnung, kann bezüglich der gleichen Resorption des Materials vorteilhaft sein.

Mit Hilfe der folgenden Beispiele wird das Herstellungsverfahren dargestellt. Weiterhin werden die hervorragenden Eigenschaften des erfindungsgemäß hergestellten Nahtmaterials im Vergleich zu herkömmlichen Nahtmaterialen auf Polymerbasis dargelegt. Es werden die kommerziellen resorbierbaren Polyglacin 910 (Ethicon VICRYL, Johnson+Johnson Intl), Polyglycolic acid, (SAFIL BRAUN Aesculap AG&CO. KG) und nicht resorbierbare Nahtmaterial Polyester (Ethicon MERSILENE Johnson+Johnson Intl.) mit erfindungsgemäß hergestellten Drähten aus einer MgAlLi-Legierung, MGAl3Li9, bezüglich der mechanischen Kennwerte nach der Prüfrichtung Zugversuch überprüft und verglichen.

Die Untersuchung wurde mittels einer universellen Prüfmaschine Zwick 10kN bei der durchgeführt. Die Zugparameter waren für alle acht geprüften Materialien konstant: Vorkraft 10N, die Prüfgeschwindigkeit 5mm/min, sowie die Raumtemperatur 20°C und die Raumfeuchtigkeit 50 %. Die kommerziellen Nahtmaterialien wurden ohne Knoten als auch mit Knoten überprüft. Theoretisch werden die Festigkeiten für die Nähte, die mit dem Knoten r versehen sind, um den Faktor 50 niedriger als für die Materialien ohne Knoten; die Untersuchung hat diese Tendenz bewiesen.

In einem geometrischen Vergleich, der für die derartige Prüfung relevant ist, stehen die geprüften Nahtmaterialien aus Magnesium in einer fünffachen Überdimension zu der Polymergruppe. Aus dem Vergleichsresultat lassen sich jedoch klare Möglichkeiten für das Material mit einem Rundprofil mit einem Durchmesser von ca. 1 mm aus Magnesiumwerkstoff ziehen.

Wie aus der Tabelle 1 zu entnehmen ist, haben die Magnesiumwerkstoffdrähte ohne Wärmebehandlung als auch nach der Wärmebehandlung (als einen Vorbereitungsschritt für eine weitere Umformphase) für F max einen Wert von 200N weit überschritten.

**Tabelle 1**

| Probe | F max N | ΔL max mm |
|---|---|---|
| Mersilene Johnson ohne Knoten L0 = 100mm | 80,09 | 35,99 |
| Vicryl Johnson ohne Knoten L0 = 100mm | 22,36 | 28,84 |
| Vicryl Johnson ohne Knoten L0 = 100mm | 22,63 | 28,37 |
| Braun Safil mit Knoten L0 = 100mm | 23,21 | 21,45 |
| Vicryl Johnson mit Knoten L0 = 100mm | 11,86 | 4,29 |
| Vicryl Johnson mit Knoten L0 = 100mm | 13,69 | 7,61 |
| MgAlLi ohne Knoten L0 = 100mm | 261,34 | 17,03 |
| MgAlLi ohne Knoten mit Wärmebehandlung L0 = 100mm | 213,91 | 18,76 |

## Patentansprüche

1. Verfahren zur Herstellung eines Elements aus einem Magnesiumwerkstoff, umfassend die Schritte:
a) Warmstrangpressen des Magnesiumwerkstoffs unter Erhalt eines Strangmaterials;
b) Wärmebehandeln des im Schritt a) erhaltenen Strangmaterials;
c) Ziehen des im Schritt b) wärmebehandelten Strangmaterials unter Erhalt eines Halbzeugs;
d) Legen einer Litze umfassend mindestens zwei der im Schritt c) erhaltenen Halbzeuge;
e) Ziehen der im Schritt d) gelegten Litze, wobei dieses Ziehen ein Warmziehen und/oder Kaltziehen ist, und
f) abschließendes Kaltziehen der im Schritt e) erhaltenen Litze.

2. Verfahren nach Anspruch 1, wobei der Durchmesser des Elements nach Schritt e) und/oder des Halbzeugs nach Schritt c) 0,5 mm oder kleiner ist, bevorzugt ist der Durchmesser des Elements nach Schritt e) und/oder des Halbzeuges nach Schritt c) 0,3 mm oder kleiner.

3. Verfahren nach einem der Ansprüche 1 oder 2, wobei im Schritt c) das Ziehen mindestens ein Kaltziehen, eine Wärmebehandlung des kaltgezogenen Halbzeugs und mindestens einmal ein Warmziehen des Halbzeugs in dieser Reihenfolge umfasst.

4. Verfahren nach einem der vorherigen Ansprüche, **dadurch gekennzeichnet, dass** das Ziehen gemäß Schritt e) unter Ausschluss von Sauerstoff erfolgt, bevorzugt erfolgt das Ziehen in Schritt e) unter Schutzgas.

5. Verfahren nach Anspruch 4, **dadurch gekennzeichnet, dass** das dritte Ziehen gemäß Schritt f) unter Sauerstoffausschluss, bevorzugt unter Schutzgas, erfolgt.

6. Verfahren nach einem der vorherigen Ansprüche, **dadurch gekennzeichnet, dass** im Schritt d) mindestens 4 Halbzeuge eines Magnesiumwerkstoffs zusammengelegt und gegebenenfalls gedreht werden.

7. Verfahren nach einem der vorherigen Ansprüche, **dadurch gekennzeichnet, dass** im Schritt des Ziehens gemäß Schritt c) oder e) eine Verringerung des Durchmesser des Halbzeuges bzw. der Litze um höchstens 0,05 mm erfolgt.

8. Verfahren nach einem der vorherigen Ansprüche, **dadurch gekennzeichnet, dass** im Schritt e) die zu ziehende Litze und der Ziehstein erwärmt werden.

9. Verfahren nach einem der vorherigen Ansprüche, **dadurch gekennzeichnet, dass** im Schritt c) die Schritte des Erwärmens des Halbzeugs und des Kaltziehen des Halbzeugs mindestens zweimal, bevorzugt mindestens viermal, wiederholt werden.

10. Verfahren nach einem der vorherigen Ansprüche, **dadurch gekennzeichnet, dass** im Schritt c) das Halbzeug mindestens einmal erwärmt und anschließend über einen warmen Ziehstein gezogen wird, bevorzugt erfolgt der Schritt des Erwärmens des Halbzeugs und des Warmziehens unter Schutzgas, insbesondere wird der Schritt des Erwärmens und Warmziehens des Halbzeugs mindestens einmal wiederholt.

11. Verfahren nach einem der vorherigen Ansprüche, wobei das Element ein resorbierbares Drahtmaterial ist.

12. Verfahren nach einem der vorherigen Ansprüche, **dadurch gekennzeichnet, dass** das Verfahren eines zur Herstellung von resorbierbarem Nahtmaterial aus einem Magnesiumwerkstoff ist.

13. Element aus einem Magnesiumwerkstoff erhalten nach einem der Verfahren 1 bis 12.

14. Element nach Anspruch 13, wobei es sich um ein resorbierbares Nahtmaterial handelt.

15. Element nach einem der Ansprüche 13 oder 14, **dadurch gekennzeichnet, dass** es eine Zugfestigkeit von 110-390 N/mm² aufweist.

16. Element nach einem der Ansprüche 13 bis 15, wobei der Magnesiumwerkstoff ein Werkstoff ist, der neben Mg auch Al und Li enthält, bevorzugt enthält der Magnesiumwerkstoff weiterhin mindestens ein Element ausgewählt aus der Gruppe aus Zr, Mn, Seltene Erden und Zn.

17. Element nach einem der Ansprüche 13 bis 16, wobei der Magnesiumwerkstoff eine Magnesiumlegierung ist.

## Claims

1. A process to create an element from magnesium substance, comprising the following steps:
a) Hot extrusion of the magnesium substance, yielding the cord material;
b) Thermal treatment of the cord material obtained in step a);
c) Drawing of the thermally-treated cord material from point b), yielding the semi-finished product;
d) Laying of a litz wire comprising of at least two semi-finished products from point c);
e) Drawing of the litz wire from point d), whereby the mode of the drawing is either hot or cold drawing; and
f) Final cold drawing of the litz wire produced in point e).

2. A process occurring in accordance with claim 1, whereby the diameter of the element after step e) and/or of the semi-finished product after step c) is 0.5mm or below, while it is preferred that the diameter of the element after step e) and/or the semi-finished product after step c) is 0.3 mm or smaller.

3. A process occurring in accordance with claim 1 or 2, whereby the drawing process referred to in step c) comprises at least one instance of cold drawing, one of thermal treatment of the semi-finished product after it has been cold-drawn and at least one of hot drawing of the semi-finished product, in that order.

4. A process occurring in accordance with one of the claims above, whose characteristic feature is the fact that the drawing process in step e) occurs in an anaerobic environment, preferably in an inert atmosphere.

5. A process occurring in accordance with claim 4, whose characteristic feature is the fact that the third drawing in step f) occurs in an anaerobic environment, preferably in an inert atmosphere.

6. A process occurring in accordance with one of the claims above, whose characteristic feature is the fact that at least 4 semi-finished products made of magnesium substance are used in point d) and drawn, if necessary.

7. A process occurring in accordance with one of the claims above, whose characteristic feature is the fact that the diameter of the semi-finished product or the litz wire decreases by 0.05mm at the most in the course of the drawing process envisioned in points c) or e).

8. A process occurring in accordance with one of the previous claims, whose characteristic feature is the fact that the draw plate and the litz wire drawn in step e) are heated.

9. A process occurring in accordance with one of the claims above, whose characteristic feature is the fact that the warming of the semi-finished product and the cold drawing of the semi-finished product set out in step c) occur at least twice, and preferably four times.

10. A process occurring in accordance with one of the claims above, whose characteristic feature is the fact that the semi-finished product is heated at least once and finally drawn on a hot draw plate, whereby the heating of the semi-finished product and the hot drawing are done in an inert atmosphere, and in particular the steps of heating and hot drawing of the semi-finished product are repeated at least once.

11. A process occurring in accordance with one of the claims above, whose characteristic feature is the fact that the element is an absorbable wire material.

12. A process occurring in accordance with one of the claims above, whose characteristic feature is the fact that the procedure is used to create an absorbable suture material from the magnesium substance.

13. Production of an element made of magnesium substance by using procedures set out in 1 through 12.

14. An element in accordance with claim 13, whereby the element is an absorbable suture material.

15. An element in accordance with claim 13 or 14, whose characteristic feature is its tensile strength of 110-390 N/mm².

16. An element in accordance with one of the claims 13-15, whereby the magnesium substance is a material containing, apart from Mg, also Al and Li, and preferably additionally one more element selected from the group of Zr, Mn, rare earth elements and Zn.

17. An element in accordance with one of the claims 13-16, whereby the magnesium substance is a magnesium alloy.

## Revendications

1. Procédé de fabrication d'un élément en un matériau à base de magnésium, comprenant les étapes :
a) Extrusion à chaud du matériau à base de magnésium pour obtenir un matériau de bande ;
b) Traitement thermique du matériau de bande obtenu à l'étape a) ;
c) Étirage du matériau de bande traité thermiquement à l'étape b) pour obtenir un produit semi-fini ;
d) Mise en forme d'un toron comprenant au moins deux produits semi-finis obtenus à l'étape c) ;
e) Étirage du toron obtenu à l'étape d), sachant que cet étirage est un étirage à chaud et/ou à froid, et
f) étirage à froid final du toron obtenu à l'étape e).

2. Procédé prévu par la revendication 1, sachant que l'élément, conformément à l'étape e), et/ou le produit semi-fini, conformément à l'étape c), présente un diamètre inférieur ou égal à 0,5 mm, l'élément, conformément à l'étape e), et/ou le produit semi-fini, conformément à l'étape c), présente de préférence un diamètre inférieur ou égal à 0,3 mm.

3. Procédé prévu par la revendication 1 ou 2, sachant que l'étirage à l'étape c) comprend au moins un étirage à froid, un traitement thermique du produit semi-fini tiré à froid et un étirage à chaud de ce produit semi-fini au moins dans cet ordre.

4. Procédé prévu par une des revendications précédentes, **caractérisé par le fait que** l'étirage, conformément à l'étape e), est effectué en absence d'oxygène, l'étirage est effectué à l'étape e) de préférence sous gaz de protection.

5. Procédé prévu par la revendication 4, **caractérisé par le fait que** le troisième étirage, conformément à l'étape f), est effectué en absence d'oxygène, de préférence, sous gaz de protection.

6. Procédé prévu par une des revendications précédentes, **caractérisé par le fait que** 4 produits semi-finis au moins d'un matériau à base de magnésium sont réunis à l'étape d) et éventuellement toronnés.

7. Procédé prévu par une des revendications précédentes, **caractérisé par le fait que** le diamètre du produit semi-fini ou du toron est réduit de 0,05 mm au plus à l'étape de l'étirage c) ou e).

8. Procédé prévu par une des revendications précédentes, **caractérisé par le fait que** le toron qui doit être soumis à l'étirage et la filière sont chauffés à l'étape e).

9. Procédé prévu par une des revendications précédentes, **caractérisé par le fait que** les étapes d'échauffement du produit semi-fini et l'étirage à froid du produit semi-fini sont effectués à l'étape c) au moins à deux reprises, de préférence à quatre reprises au moins.

10. Procédé prévu par une des revendications précédentes, **caractérisé par le fait que** le produit semi-fini est chauffé à l'étape c) au moins une fois et ensuite tiré à travers une filière chaude, de préférence, cet étape de l'échauffement du produit semi-fini et de l'étirage à chaud est effectué sous gaz de protection, en particulier, l'étape de l'échauffement et de l'étirage à chaud du produit semi-fini est effectué à une reprise au moins.

11. Procédé prévu par une des revendications précédentes, **caractérisé par le fait que** l'élément est un matériau sous forme d'un fil résorbable.

12. Procédé prévu par une des revendications précédentes, **caractérisé par le fait que** c'est un procédé de fabrication d'un matériau de suture résorbable en un matériau à base de magnésium.

13. Élément en un matériau à base de magnésium obtenu par un procédé de 1 à 12

14. Élément conforme à la revendication 13, sachant il s'agit d'un matériau de suture résorbable.

15. Élément prévu par une des revendications précédentes 13 ou 14, **caractérisé par le fait qu'**il présente une résistance à la traction de 110-390 N/mm.

16. Élément prévu par une des revendications précédentes de 13 à 15, sachant que le matériau à base de magnésium est un matériau qui contient, indépendamment du Mg, aussi l'Al et le Li, le matériau à base de magnésium contient de préférence en plus au moins un élément choisi dans le groupe constitué par Zr, Mn, des terres rares et Zn.

17. Élément prévu par une des revendications précédentes de 13 à 16, sachant que le matériau à base de magnésium est un alliage de magnésium.
